(19) **Europäisches Patentamt / European Patent Office / Office européen des brevets**

(11) **EP 4 779 533 A1**

(12) **EUROPEAN PATENT APPLICATION**
published in accordance with Art. 153(4) EPC

(43) Date of publication:
**22.07.2026 Bulletin 2026/30**

(21) Application number: **23952312.9**

(22) Date of filing: **15.09.2023**

(51) International Patent Classification (IPC):
*G06N 20/00* (2019.01)

(52) Cooperative Patent Classification (CPC):
**G06N 20/00**

(86) International application number:
**PCT/JP2023/033775**

(87) International publication number:
**WO 2025/057424 (20.03.2025 Gazette 2025/12)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **FUJITSU LIMITED**
**Kawasaki-shi, Kanagawa 211-8588 (JP)**

(72) Inventors:
- **ISHIZAKI, Ryo**
  **Kawasaki-shi, Kanagawa 211-8588 (JP)**
- **KITAJIMA, Masato**
  **Kawasaki-shi, Kanagawa 211-8588 (JP)**
- **KURIBAYASHI, Sotaro**
  **Kawasaki-shi, Kanagawa 211-8588 (JP)**
- **HIGUCHI, Hiroyuki**
  **Kawasaki-shi, Kanagawa 211-8588 (JP)**

(74) Representative: **Hoffmann Eitle**
**Patent- und Rechtsanwälte PartmbB**
**Arabellastraße 30**
**81925 München (DE)**

(54) **INFORMATION PROCESSING PROGRAM, INFORMATION PROCESSING DEVICE, AND INFORMATION PROCESSING METHOD**

(57) An Information processing program causing a computer to perform a process including: in classification processing on input graph structure data using a machine learning model (110), acquiring a contribution degree in the classification processing for each of a plurality of partial regions included in graph structure data; and determining an evaluation for the machine learning model (110) based on similarity between the contribution degree and designation information for the partial region of the graph structure data.

FIG.1

**Description**

[Technical Field]

The present embodiment relates to an information processing program, an information processing device, and an information processing method.

[Background Art]

With the progress of computers, the use of machine learning for large-scale protein three-dimensional structure information has progressed. If discrimination (multi-value classification) between the types of virus strains (antigen cluster) can be performed based on three-dimensional structure information, it is expected that analysis of virus mutations needed for vaccine development can be advanced.

In the classification prediction issue for antigen clusters of virus strains, it is becoming possible to perform classification prediction with higher accuracy by using three-dimensional structure information.

Furthermore, it is expected to advance the analysis of virus mutations by analyzing the classification prediction results in detail.

Therefore, it has been desired to construct a machine learning model of which the reason for determining the classification prediction result is more easily interpreted.

For example, protein three-dimensional structure information is converted into graph data, and a machine learning model is created based on the created graph data.

Conventionally, a plurality of methods are prepared to express input graph data in combination with information such as three-dimensional structure information as a graph model, the prepared plurality of methods are used as candidates, and the candidates are compared using a classification prediction accuracy degree index. Then, a criterion is used in which the candidate with the highest accuracy degree is selected from among these candidates, and the selected candidate is regarded as a graph model that can be easily interpreted.

[Prior Art Reference]

[Patent Document]

[Patent Document 1]
Japanese Laid-Open Patent Publication No. 2019-159432
[Patent Document 2]
US Patent No. 11176462
[Patent Document 3]
International Publication Pamphlet No. WO 2018/052131
[Patent Document 4]
Japanese National Publication of International Patent Application No. 2022-536343
[Patent Document 5]
Japanese National Publication of International Patent Application No. 2018-506963
[Patent Document 6]
US Patent Application Publication No. 2022/0076783

[Summary of Invention]

[Problems to be Solved by Invention]

However, a machine learning model having a high classification accuracy degree is not necessarily a machine learning model that is easy to interpret, and in that case, there is a problem that a machine learning model that is easy to interpret is not selected.

In one aspect, an object of the present embodiment is to select a machine learning model taking into consi deration not only a classification accuracy degree but also ease of interpretation.

[Means to Solve the Problem]

According to an aspect of the embodiments, An Information processing program causing a computer to perform a process including: in classification processing on input graph structure data using a machine learning model, acquiring a contribution degree in the classification processing for each of a plurality of partial regions included in graph structure data; and determining an evaluation for the machine learning model based on similarity between the contribution degree and designation information for the partial region of the graph structure data.

[Effect of Invention]

According to an embodiment, it is possible to select a machine learning model taking into consideration ease of interpretation.

[Brief Description of Drawings]

Fig. 1 is a diagram schematically illustrating a configuration of an information processing device according to an embodiment;
Fig. 2 is a block diagram illustrating a hardware (HW) configuration example of a computer that realizes functions of the information processing device according to an embodiment;
Fig. 3 is a diagram exemplifying explanation result information in the information processing device according to an embodiment;
Fig. 4 is a diagram exemplifying protein position information in the information processing device according to an embodiment;
Fig. 5 is a diagram for explaining processing of a matching index value calculation unit of the information processing device according to an embodiment;
Fig. 6 is a diagram for explaining processing of a graph selection unit of the information processing device according to an embodiment; and
Fig. 7 is a flowchart for explaining processing of the matching index value calculation unit and the graph selection unit of the information processing device according to an embodiment.

[Description of Embodiments]

Hereinafter, an embodiment of the present information processing program, information processing device, and information processing method will be described with reference to the drawings. However, the embodiment described below is merely an example, and it is not intended to exclude the application of various modifications and technologies that are not explicitly described in the embodiment. That is, the present embodiment can be variously modified and implemented without departing from the gist thereof. Each drawing is not intended to include only the components illustrated in the drawing, but may include other functions and the like.

(A) Configuration

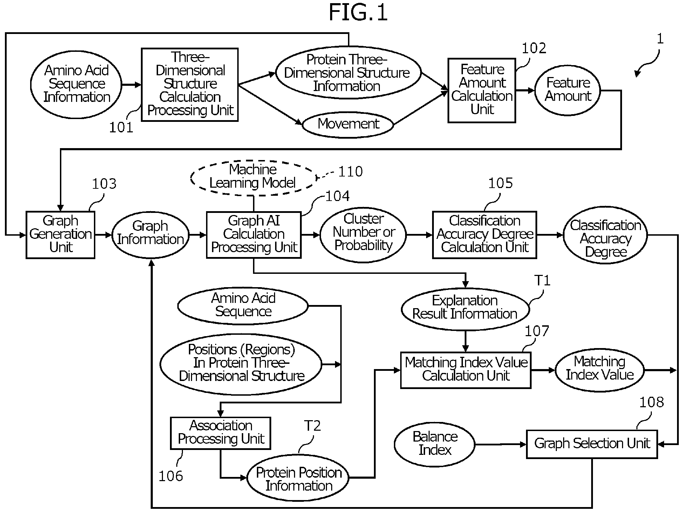

Fig. 1 is a diagram schematically illustrating a configuration of an information processing device 1 according to an embodiment.
The present information processing device 1 performs training (machine learning) of a machine learning model that predicts the type of virus strain (antigen cluster) from an amino acid sequence constituting a protein of a virus (training phase).
In the training phase, in the present information processing device 1, an amino acid sequence constituting a protein of a virus is input, and an antigen cluster name of the virus is used as correct data.
Furthermore, the present information processing device 1 predicts (infers) an antigen cluster of a virus by using the trained machine learning model (prediction phase).
In the prediction phase, in the present information processing device 1, an amino acid sequence constituting a protein of a virus is input, and the machine learning model predicts an antigen cluster name of the virus.

(A-1) Hardware Configuration Example

The functions of the information processing device 1 according to an embodiment may be realized by one computer, or may be realized by two or more computers. Furthermore, at least some of the functions of the information processing device 1 may be realized by using hardware (HW) resources and network (NW) resources provided by a cloud environment.

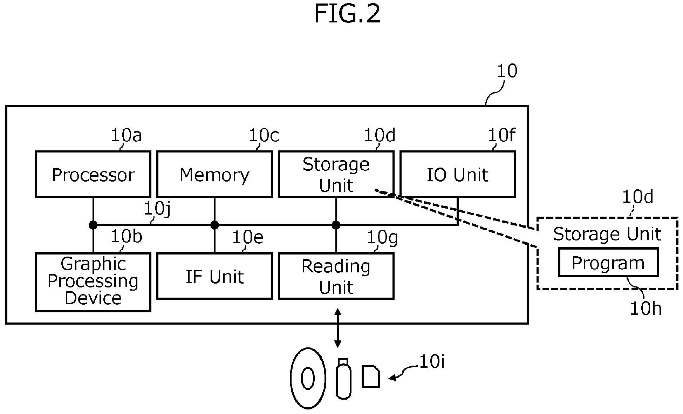

Fig. 2 is a block diagram illustrating a hardware (HW) configuration example of a computer 10 that realizes the functions of the information processing device 1 according to an embodiment. In a case where a plurality of computers are used as HW resources for realizing the functions of the information processing device 1, each computer may have the HW configuration illustrated in Fig. 2.

As illustrated in Fig. 2, the computer 10 may include, for example, a processor 10a, a graphic processing device 10b, a memory 10c, a storage unit 10d, an interface (IF) unit 10e, an input/output (IO) unit 10f, and a reading unit 10g as a HW configuration.

The processor 10a is an example of an arithmetic processing device that performs various controls and computations, and is a controller that executes various processes. The processor 10a may be communicably connected to each block in the computer 10 via a bus 10j. Note that the processor 10a may be a multiprocessor including a plurality of processors, may be a multi-core processor including a plurality of processor cores, or may have a configuration including a plurality of multi-core processors.

The processor 10a may be, for example, an integrated circuit (IC) such as a CPU, an MPU, an APU, a DSP, an ASIC, or an FPGA. Note that a combination of two or more of these integrated circuits may be used as the processor 10a. CPU is an abbreviation for Central Processing Unit, and MPU is an abbreviation for Micro Processing Unit. APU is an abbreviation for Accelerated Processing Unit. DSP is an abbreviation for Digital Signal Processor, ASIC is an abbreviation for Application Specific IC, and FPGA is an abbreviation for Field-Programmable Gate Array.

The graphic processing device 10b performs screen display control on an output device such as a monitor in the IO unit 10f. The graphic processing device 10b may have a configuration as an accelerator that executes machine learning processing and prediction processing using a machine learning model. The graphic processing device 10b may be any of various arithmetic processing devices, for example, an integrated circuit (IC) such as a graphics processing unit (GPU), an APU, a DSP, an ASIC, or an FPGA.

The memory 10c is an example of HW that stores various types of information such as data and programs. The memory 10c may be, for example, one or both of a volatile memory such as a dynamic random access memory (DRAM) and a nonvolatile memory such as a persistent memory (PM).

The storage unit 10d is an example of HW that stores various types of information such as data and programs. The storage unit 10d may be any of various storage devices, for example, a magnetic disk device such as a hard disk drive (HDD), a semiconductor drive device such as a solid state drive (SSD), and a nonvolatile memory. The nonvolatile memory may be, for example, a flash memory, a storage class memory (SCM), or a read only memory (ROM).

The storage unit 10d may store a program 10h (information processing program) that realizes all or some of the various functions of the computer 10.

For example, the processor 10a of the information processing device 1 can realize a function in the training phase and a function in the prediction phase, which will be described later, by loading the program 10h stored in the storage unit 10d into the memory 10c and executing the program 10h.

The IF unit 10e is an example of a communication IF that controls connection and communication between the computer 10 and other computers. For example, the IF unit 10e may include an adapter conforming to local area network (LAN) such as Ethernet®, optical communication such as fibre channel (FC), or the like. The adapter may support one or both of wireless and wired communication systems.

For example, the computer 10 may be communicably connected to other computers and databases (not illustrated) via the IF unit 10e and the network. Note that the program 10h may be downloaded from the network to the computer 10 via the communication IF and stored in the storage unit 10d.

The IO unit 10f may include one or both of an input device and an output device. The input device may be, for example, a keyboard, a mouse, or a touch panel. The output device may be, for example, a monitor, a projector, or a printer. In addition, the IO unit 10f may include a touch panel or the like in which the input device and the output device are integrated. The output device may be connected to the graphic processing device 10b.

The reading unit 10g is an example of a reader that reads out data and program information recorded on a recording medium 10i. The reading unit 10g may include a connection terminal or device to which the recording medium 10i can be connected or inserted. The reading unit 10g may be, for example, an adapter conforming to a universal serial bus (USB) or the like, a drive device that accesses a recording disk, and a card reader that accesses a flash memory such as an SD card. Note that the program 10h may be stored in the recording medium 10i, and the reading unit 10g may read out the program 10h from the recording medium 10i and store the program 10h in the storage unit 10d.

The recording medium 10i may be, for example, a nontransitory computer-readable recording medium such as a magnetic/optical disk or a flash memory. The magnetic/optical disk may be, for example, a flexible disk, a compact disc (CD), a digital versatile disc (DVD), a Bluray disc, or a holographic versatile disc (HVD). The flash memory may be, for example, a semiconductor memory such as a USB memory or an SD card.

The above-described HW configuration of the computer 10 is an example. Therefore, the HW in the computer 10 may be increased or decreased (for example, by adding or deleting an optional block), divided, or integrated in any combination, or buses may be added or deleted as appropriate.

(A-2) Functional Configuration Example

As illustrated in Fig. 1, the information processing device 1 may have, for example, functions as a three-dimensional structure calculation processing unit 101, a feature amount calculation unit 102, a graph generation unit 103, a graph AI calculation processing unit 104, a classification accuracy degree calculation unit 105, an association processing unit 106, a matching index value calculation unit 107, a graph selection unit 108, and a machine learning model 110. These functions may be realized by the hardware of the computer 10 (see Fig. 2).

The three-dimensional structure calculation processing unit 101 analyzes a three-dimensional structure of a protein of a virus. When an amino acid sequence (amino acid sequence information) constituting a protein of a virus is input, the three-dimensional structure calculation processing unit 101 analyzes a three-dimensional structure of the protein. The three-dimensional structure calculation processing unit 101 outputs protein three-dimensional structure information as an analysis result. The protein three-dimensional structure information may include, for example, coordinates for each atom of each amino acid as information indicating the three-dimensional structure, or may include information indicating a movement of a molecule.

The function as the three-dimensional structure calculation processing unit 101 may be realized using a known protein structure calculation tool. As a protein structure calculation tool, for example, a protein three-dimensional structure prediction tool such as AlphaFold2 may be used. The three-dimensional structure calculation processing unit 101 may output a result of searching databases in which protein three-dimensional structure information experimentally determined by, for example, X-ray crystallography, nuclear magnetic resonance (NMR), singleparticle analysis using a cryo-electron microscope, or electron diffraction has been registered in advance. Furthermore, the three-dimensional structure calculation processing unit 101 may perform, for example, molecular dynamics (MD) simulations using the three-dimensional structure information output from these databases as initial values.

In the protein three-dimensional structure information, for example, a coordinate value of each amino acid or information indicating a movement of a molecule may be associated with identification information for identifying a virus.

The protein three-dimensional structure information output by the three-dimensional structure calculation processing unit 101 may be stored in a predetermined storage area or the like of the memory 10c or the storage unit 10d.

The feature amount calculation unit 102 generates a three-dimensional structure feature amount based on the protein three-dimensional structure information created by the three-dimensional structure calculation processing unit 101. The three-dimensional structure feature amount indicates a feature amount of the three-dimensional structure.

The feature amount calculation unit 102 may generate the feature amount of the three-dimensional structure using a known feature amount conversion technique. For example, the feature amount calculation unit 102 may perform the feature amount conversion using a statistical technique such as support vector regression (SVR), neural network (NN), or principal component analysis (PCA). Alternatively, the feature amount calculation unit 102 may discretize categorical variables using knowledge of experts familiar with the field of virology.

The three-dimensional structure feature amount calculated by the feature amount calculation unit 102 may be stored in a predetermined storage area or the like of the memory 10c or the storage unit 10d.

The graph generation unit 103 creates graph information based on the protein three-dimensional structure information generated by the three-dimensional structure calculation processing unit 101 and the three-dimensional structure feature amount generated by the feature amount calculation unit 102. The graph information may be regarded as graph data. The graph information is an example of graph structure data.

The graph generation unit 103 may convert information regarding a plurality of viruses included in the graph information into data in a format that can be processed by the machine learning model 110.

For example, the graph generation unit 103 may create a plurality of types of graph information from one protein three-dimensional structure by appropriately changing a graph creation parameter such as a distance between amino acids.

The graph AI calculation processing unit 104 creates the machine learning model 110 based on the graph information. In the training phase, the graph AI calculation processing unit 104 performs training (machine learning) of the machine learning model 110. The machine learning model 110 may be regarded as graph AI.

Here, the machine learning model 110 performs graphbased relationship training, and realizes graph classification (class classification: classification processing).

The graph includes a set of nodes and a set of edges between the nodes. The graph can be said to be a mathematical model characterized by nodes and edges.

When the graph is applied to a virus, amino acids correspond to nodes, and bonds between the amino acids correspond to edges. The bonds between the amino acids may be, for example, peptide bonds, or other types of bonds such as bonds formed by electrostatic forces.

The machine learning model 110 performs graph classification based on these graphs and edge information. At this time, the machine learning model 110 may use, as explanatory variables, a set of nodes in the graph information created by the graph generation unit 103, a set of edges between the nodes included in the node set, and a set of attributes of the nodes included in the node set. In addition, the machine learning model 110 may use a cluster number corresponding to an antigen cluster as an objective variable.

In the graph classification, a parameter for each node may be used as a node attribute to serve as a clue for classification. The machine learning model 110 may output a cluster number or a probability obtained by classifying the protein three-dimensional structure as a result of graph classification.

In order to cause the machine learning model 110 to perform graph classification, an edge may be explicitly given to the machine learning model 110. Therefore, the graph AI calculation processing unit 104 defines that amino acids that are adjacent to each other based on the amino acid sequence have an edge therebetween. In addition, amino acids that are within a certain distance range due to electrostatic forces or the like may also be defined as having an edge therebetween.

The function as the machine learning model 110 can be realized using a known technique. For example, the function as the machine learning model 110 may be realized by Deep Tensor®.

Based on the graph information, the graph AI calculation processing unit 104 may create information to be input to the machine learning model 110 (information for graph AI input) by arranging, for each edge of the amino acid sequence constituting the virus, respective attributes of two amino acids to which the edge is bound in units of bond. The two amino acids to which the edge is bound may be regarded as an amino acid pair. In the amino acid pair, the amino acid that is the start point of the edge may be regarded as a start node, and the amino acid that is the end point of the edge may be regarded as an end node.

The machine learning model 110 may be a deep neural network (DNN) including a plurality of hidden layers between an input layer and an output layer.

For example, the NN performs forward processing (forward propagation processing) in which input data is input into an input layer and predetermined calculations are sequentially executed in hidden layers composed of convolution layers, pooling layers, and the like, thereby sequentially transmitting information obtained by the computations from an input side to an output side. After the execution of the forward processing, backward processing (backward propagation processing) for determining parameters to be used in the forward processing is executed in order to minimize a value of an error function obtained from output data (graph classification result) output from an output layer and correct data (cluster name). Then, update processing is executed to update variables such as weights based on the result of the backward propagation processing. For example, a gradient descent method may be used as an algorithm for determining an update width for the weights used in the backward propagation processing.

In addition, in the prediction phase, the graph AI calculation processing unit 104 causes the machine learning model 110 to perform graph classification using the information for graph AI input as input data, and for example, predict (infer) a cluster number or predict a probability of a cluster number.

The graph AI calculation processing unit 104 may input the feature amount (three-dimensional structure feature amount) related to the three-dimensional structure of the protein of the virus to the machine learning model 110, causing the machine learning model 110 to predict a cluster number.

In addition, in each of the training phase and the prediction phase, the graph AI calculation processing unit 104 inputs the information for graph AI input to the machine learning model 110, causing the machine learning model 110 to perform graph classification (class classification), and then create prediction basis information.

The prediction basis information may be, for example, a contribution degree (a prediction contribution degree) for obtaining a prediction result when the machine learning model 110 performs graph classification. The graph AI calculation processing unit 104 obtains prediction basis information for each amino acid included in the virus.

The graph AI calculation processing unit 104 may calculate the prediction basis information using an explainable AI technology such as Deep Tensor. In the present information processing device 1, the explainable AI technology particularly has functions of identifying an important portion that forms a reason (basis) for prediction with respect to components (partial regions) of input data expressing prediction target information, and outputting a value according to its importance.

The components (partial regions) of input data expressing prediction target information may be nodes or edges of graph data. In the present embodiment, an example will be described in which components (partial regions) of input data expressing prediction target information are nodes.

Using the graph data expressing protein information as input data, the graph AI calculation processing unit 104 outputs a prediction contribution degree for a node that is a component (partial region) of the graph data. Here, the prediction contribution degree is calculated by constructing a training process for creating a machine learning model 110 that better classifies antigen clusters and performing inference on the prediction target using the machine learning model 110.

In addition, the graph AI calculation processing unit 104 creates explanation result information using the prediction basis information.

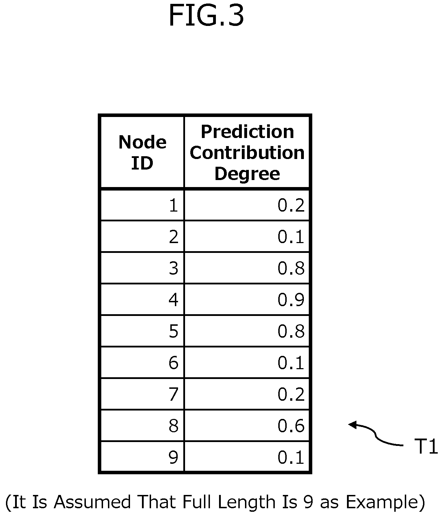

Fig. 3 is a diagram exemplifying explanation result information in the information processing device 1 according to an embodiment.

The explanation result information illustrated in Fig. 3 associates a node ID indicating the entity of a node with a prediction contribution degree. Fig. 3 illustrates an example in which it is assumed that the full length is 9. In addition, in Fig. 3, sorting is performed based on the node IDs. The node IDs may be regarded as positions in the protein three-dimensional structure, or may be simply referred to as positions. The node IDs are information for identifying a plurality of partial regions included in the graph structure data.

In Fig. 3, the explanation result information is indicated by reference sign T1. Hereinafter, it may be referred to as explanation result information T1.

The graph AI calculation processing unit 104 acquires the node ID and the prediction contribution degree based on the prediction basis information, and associates them to create the explanation result information T1. The explanation result information T1 indicates positions that are identified as having high contribution degrees by the explanation function of the graph machine learning of the graph AI calculation processing unit 104.

The graph AI calculation processing unit 104 creates explanation result information T1 for each sample (input protein three-dimensional structure). In addition, the graph AI calculation processing unit 104 creates explanation result information T1 for each of the plurality of types of machine learning models 110.

In the classification processing on the input graph data (graph structure data) by the machine learning model 110, the graph AI calculation processing unit 104 creates a contribution degree (prediction contribution degree and explanation result information T1) in the classification processing for each of the plurality of nodes or edges (partial regions) included in the graph data.

The explanation result information T1 created by the graph AI calculation processing unit 104 may be stored in a predetermined storage area or the like of the memory 10c or the storage unit 10d.

In the machine learning model 110 (graph AI), the contribution degree is obtained for each three-dimensional structure or for each amino acid. Therefore, the graph AI calculation processing unit 104 may obtain a sample average of contribution degrees, for example, in a predetermined unit such as each cluster, year, or amino acid, and use the sample average as the prediction basis information.

The result of the prediction performed by the machine learning model 110, which is caused by the graph AI calculation processing unit 104, the values of the prediction basis information calculated by the machine learning model 110, which is caused by the graph AI calculation processing unit 104, and the like may be stored in a predetermined storage area or the like of the memory 10c or the storage unit 10d.

In the prediction phase, the classification accuracy degree calculation unit 105 calculates a classification accuracy degree (prediction accuracy degree) by comparing the cluster number classification result (prediction result) obtained by the machine learning model 110 with the correct data. The classification accuracy degree calculated by the classification accuracy degree calculation unit 105 may be stored in a predetermined storage area or the like of the memory 10c or the storage unit 10d.

The association processing unit 106 creates protein position information. The protein position information is information indicating positions (regions) in an amino acid sequence (protein three-dimensional structure), and indicates positions (regions) where the features for classifying antigen clusters are particularly prominent. The protein position information may be regarded as indicating positions (regions) that are known by experts familiar with the field of virology as positions (regions) particularly related to (having a large influence on) the classification of antigen clusters. The positions particularly related to the classification of antigen clusters may be a set of node IDs indicating nodes that are recognized by experts as having a large influence on the classification of antigen clusters in the graph data created based on the protein three-dimensional structure. The protein position information is an example of designation information for partial regions of graph structure data.

In the protein position information, the positions (regions) particularly related to the classification of antigen clusters may be represented by a set of positions of residue numbers.

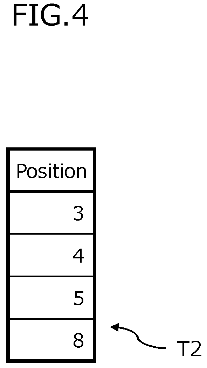

Fig. 4 is a diagram exemplifying protein position information in the information processing device 1 according to an embodiment.

In Fig. 4, the protein position information is indicated by reference sign T2. Hereinafter, it may be referred to as protein position information T2.

The protein position information T2 exemplified in Fig. 4 indicates a plurality of numerical values indicating positions having a large influence on the classification of antigen clusters in the protein three-dimensional structure. The numerical value indicating each position included in the protein position information T2 corresponds to the node ID. A process of collating the position in the protein position information with the node ID in the explanation result information may be performed.

The association processing unit 106 creates protein position information based on information regarding amino acid sequences already known by experts familiar with the virology field and information on positions (regions) in the three-dimensional structure of the protein. In addition, the association processing unit 106 may acquire the protein position information T2 created in advance by downloading or another technique.

Based on the explanation result information T1 and the protein position information T2, the matching index value calculation unit 107 calculates a matching index value indicating a matching degree between the nodes having high prediction contribution degrees in the explanation result information T1 and the nodes indicated in the protein position information T2.

The matching index value indicates a degree of matching tendencies between the explanation result information T1 (contribution degree and prediction contribution degree) and the protein position information T2 including designation information for partial regions such as nodes or edges of the graph structure data, and indicates a similarity degree therebetween. In addition, the matching index value may be regarded as a value indicating interpretability (classification interpretation) of the mechanism of prediction (classification) performed by the machine learning model 110.

The matching index value calculation unit 107 acquires the explanation result information T1 created by the graph AI calculation processing unit 104 and the protein position information T2 created by the association processing unit 106, and calculates a matching index value using the explanation result information T1 and the protein position information T2.

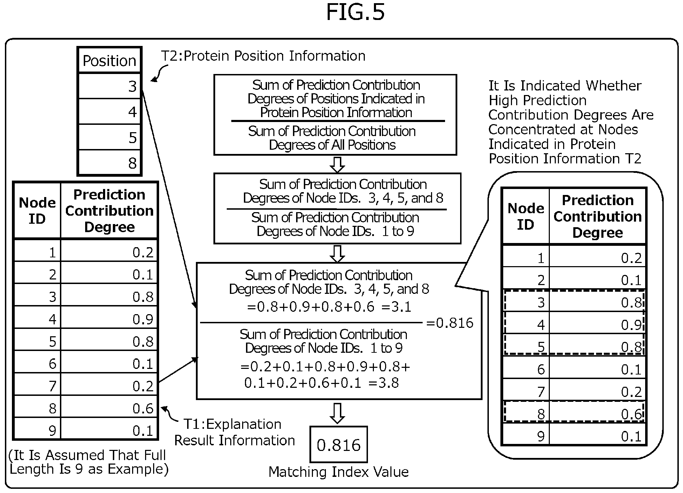

Fig. 5 is a diagram for explaining processing of the matching index value calculation unit 107 of the information processing device 1 according to an embodiment.

The matching index value calculation unit 107 calculates, as the matching index value, a ratio of "the sum of prediction contribution degrees of node IDs (positions) corresponding to all values included in the protein position information T2 among node IDs included in the explanation result information T1" to "the sum of prediction contribution degrees of all node IDs (positions) included in the explanation result information T1".

In the example illustrated in Fig. 5, values corresponding to the node IDs 3, 4, 5, and 8 are set as positions in the protein position information T2. In this case, the matching index value calculation unit 107 calculates the matching index value by calculating (the sum of the respective prediction contribution degrees of the node IDs 3, 4, 5, and 8)/(the sum of the respective prediction contribution degrees of the node IDs 1 to 9).

In the example illustrated in Fig. 5, (the sum of the respective prediction contribution degrees of the node IDs 3, 4, 5, and 8)=0.8+0.9+0.8+0.6=3.1.

In addition, (the sum of the respective prediction contribution degrees of the node IDs 1 to 9) =0.2+0.1+0.8+0.9+0.8+0.1+0.2+0.6+0.1=3.8.

Therefore, the matching index value calculation unit 107 obtains the matching index value of 0.816 by calculating 3.1/3.8=0.816.

The matching index value indicates a matching degree between the positions (node IDs) in the amino acid three-dimensional structure indicated by the protein position information T2 and the node IDs having high prediction contribution degrees calculated by the graph AI calculation processing unit 104. That is, the matching index value indicates whether the positions (node IDs) in the amino acid three-dimensional structure indicated by the protein position information T2 are similar to the node IDs having high prediction contribution degrees calculated by the graph AI calculation processing unit 104. In addition, the matching index value may indicate whether high prediction contribution degrees are concentrated at the positions (node IDs) indicated in the protein position information T2.

In the present embodiment, the larger the matching index value (closer to 1), the higher the matching degree between the tendency indicated by the explanation result information T1 and the tendency indicated by the protein position information T2.

When the matching index value is larger than a predetermined threshold (e.g., 0.5), it may be determined that the tendency indicated by the explanation result information T1 matches the tendency indicated by the protein position information T2.

In the example illustrated in Fig. 5, all of the nodes of the node IDs 3, 4, 5, and 8 indicated in the protein position information T2 have prediction contribution degrees greater than or equal to 0.6, and it can be said that the matching degree between the positions (node IDs) in the amino acid three-dimensional structure indicated by the protein position information T2 and the node IDs having high prediction contribution degrees calculated by the graph AI calculation processing unit 104 is high.

The graph selection unit 108 calculates an evaluation value for the machine learning model 110 based on the matching index value calculated by the matching index value calculation unit 107, the classification accuracy degree calculated by the classification accuracy degree calculation unit 105, and a balance index.

The balance index indicates a degree to which each of the matching index value and the classification accuracy degree is reflected when calculating the evaluation value, and is a weight to be applied to each of the matching index value and the classification accuracy degree. The weight applied to the matching index value is represented by reference sign w1, and the weight applied to the classification accuracy degree is represented by reference sign w2.

For example, the graph selection unit 108 may calculate an evaluation value for the machine learning model 110 by calculating the following Formula (1).

Evaluation value = w1 × classification accuracy degree + w2 × matching index value ⋯ (1)                    (1)

The balance index (w1,w2) may be w1+w2=1.0. The balance index may be set in advance by a user or the like. When determining the balance index, the values of w1 and w2 may be appropriately changed in consideration of the balance between the matching index value (ease of interpretation) and the classification accuracy degree (prediction accuracy degree).

For example, in a case where the matching index value and the classification accuracy degree are treated equally, w1=0.5 and w2=0.5 may be set. In addition, in a case where it is desired to select a machine learning model 110 having high interpretability of the classification mechanism, the value of the weight w1 to be applied to the matching index value may be increased, and in a case where it is desired to select a machine learning model 110 having a high classification accuracy degree, the value of the weight w2 to be applied to the classification accuracy degree may be increased.

The graph selection unit 108 calculates an evaluation value for each of the plurality of machine learning models 110. Then, the graph selection unit 108 evaluates the machine learning model 110 based on the evaluation value. For example, the graph selection unit 108 may determine a machine learning model 110 having the highest evaluation value as an optimal machine learning model 110.

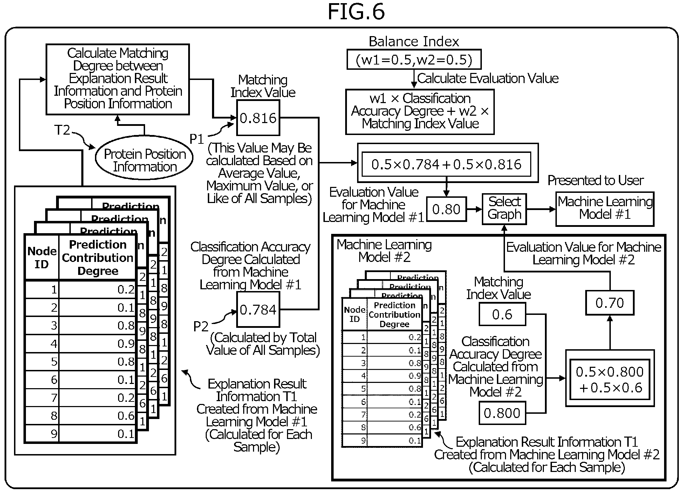

Fig. 6 is a diagram for explaining processing of the graph selection unit 108 of the information processing device 1 according to an embodiment.

The example illustrated in Fig. 6 illustrates a process in which the graph selection unit 108 compares machine learning model #1 and machine learning model #2 and selects machine learning model #1 as a machine learning model to be presented to the user.

Regarding machine learning model #1, the matching index value (see reference sign P1) calculated by the matching index value calculation unit 107 based on the explanation result information T1 and the protein position information T2 and the classification accuracy degree (see reference sign P2) calculated by the classification accuracy degree calculation unit 105 are input to the graph selection unit 108. In addition, the graph selection unit 108 acquires the balance index (w1,w2).

The graph selection unit 108 calculates an evaluation value by calculating the above Formula (1) based on the matching index value, the classification accuracy degree, and the balance index.

In the example illustrated in Fig. 6, the evaluation value for machine learning model #1 is 0.5×0.0784+0.5×0.816=0.80.

Similarly, for machine learning model #2, the graph selection unit 108 obtains the evaluation value of 0.70 by calculating the formula (0.5×0.800+0.5×0.6) based on the matching index value (0.6), the classification accuracy degree (0.800), and the balance index (0.5,0.5).

The graph selection unit 108 may compare the calculated evaluation values of the machine learning models 110 and present machine learning model #1, which has the highest evaluation value, to the user. In the example illustrated in Fig. 6, the graph selection unit 108 selects machine learning model #1, which has a large evaluation value, as a suitable machine learning model 110. It can be said that the selection of the machine learning model 110 by the graph selection unit 108 is equivalent to selecting graph information on which the machine learning model 110 is based.

The graph AI calculation processing unit 104 presents the selected machine learning model #1 to the user. The graph AI calculation processing unit 104 may present only the selected machine learning model #1 to the user.

Note that the information presented to the user by the graph selection unit 108 is not limited thereto. For example, the graph selection unit 108 may present the evaluation value for each of the plurality of machine learning models 110 to the user, and the information presented to the user by the graph selection unit 108 can be implemented in an appropriately modified manner.

(B) Operation

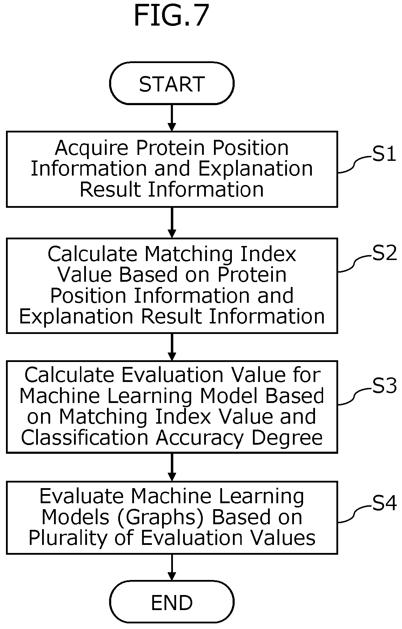

The processing of the matching index value calculation unit 107 and the graph selection unit 108 of the information processing device 1 according to an embodiment configured as described above will be described with reference to a flowchart (steps S1 to S4) illustrated in Fig. 7.

The following processing is performed in the training phase of the information processing device 1.

In step S1, the matching index value calculation unit 107 acquires the protein position information T2 and the explanation result information T1 created for each sample based on the machine learning model 110.

In step S2, the matching index value calculation unit 107 calculates a matching index value based on the explanation result information T1 and the protein position information T2.

In step S3, the graph selection unit 108 calculates an evaluation value for the machine learning model 110 based on the matching index value calculated by the matching index value calculation unit 107, the classification accuracy degree calculated by the classification accuracy degree calculation unit 105, and the balance index. The graph selection unit 108 calculates an evaluation value for each of the plurality of machine learning models 110.

In step S4, the graph selection unit 108 evaluates the machine learning models 110 based on the plurality of evaluation values. For example, the graph selection unit 108 may select a machine learning model 110 having the highest evaluation value and present the selected machine learning model 110 to the user. Furthermore, the graph selection unit 108 may present a machine learning model 110 having a high evaluation value (e.g., higher than or equal to a threshold) to the user. Thereafter, the processing ends.

(C) Effects

As described above, in the information processing device 1 according to an embodiment, based on the explanation result information T1 and the protein position information T2, the matching index value calculation unit 107 calculates a matching index value indicating a matching degree between the nodes having high prediction contribution degrees in the explanation result information T1 and the nodes indicated in the protein position information T2.

In addition, the graph selection unit 108 calculates an evaluation value for the machine learning model 110 based on the matching index value calculated by the matching index value calculation unit 107, the classification accuracy degree calculated by the classification accuracy degree calculation unit 105, and the balance index.

Then, the graph selection unit 108 evaluates the machine learning model 110 based on the calculated evaluation value.

As a result, the graph selection unit 108 can select a machine learning model 110 taking into consideration the classification accuracy degree and the ease of interpretation (good interpretability).

By setting the balance index (w1,w2) in consideration of the balance between the matching index value (ease of interpretation) and the classification accuracy degree (prediction accuracy degree), it is possible to select a machine learning model 110 that meets the user's desire in terms of the relationship between the ease of interpretation and the classification accuracy degree.

The matching index value calculation unit 107 calculates, as the matching index value, a ratio of "the sum of prediction contribution degrees of node IDs (positions) corresponding to all values included in the protein position information T2 among node IDs included in the explanation result information T1" to "the sum of prediction contribution degrees of all node IDs (positions) included in the explanation result information T1".

The graph selection unit 108 selects a machine learning model 110 of which the matching index value obtained in this manner is high, the selected machine learning model 110 calculating a high prediction contribution degree for a partial region of a protein considered to be particularly related to the classification of antigen clusters by experts familiar with the virology field. Therefore, it is possible to select a machine learning model 110 that is easy to interpret.

(D) Others

The disclosed technology is not limited to the above-described embodiment, and various modifications can be made without departing from the gist of the present embodiment. Each configuration and each process of the present embodiment can be selected as needed, or may be appropriately combined.

In the above-described embodiment, the setting of the prediction contribution degree, the setting of the protein position information, and the like are performed for the nodes in the graph data, but are not limited thereto. The setting of the prediction contribution degree, the setting of the protein position information, and the like may be performed for the edges instead of the nodes.

In addition, in the above-described embodiment, the matching index value calculation unit 107 calculates a matching index value for each sample, but the calculation of the matching index value is not limited thereto. For example, the matching index value may be calculated based on an average value, a maximum value, or the like of a plurality of samples (e.g., all samples), and the calculation of the matching index value can be performed in an appropriately modified manner.

In addition, the matching index value calculation unit 107 may calculate a proportion at which large prediction contribution degree values are concentrated at the positions set in the protein position information T2, and use the calculated rate as the matching index value (the matching degree in tendency).

Furthermore, according to the disclosure described above, the present embodiment can be carried out and manufactured by those skilled in the art.

[Reference Signs List]

1 Information processing device
10 Computer
10a Processor
10b Graphic processing device
10c Memory
10d Storage unit
10e IF unit
10f IO unit
10g Reading unit
10h Program
10i Recording medium
10j Bus
101 Three-dimensional structure feature amount processing unit
102 Feature amount calculation unit
103 Graph generation unit
104 Graph AI calculation processing unit
105 Classification accuracy degree calculation unit
106 Association processing unit
107 Matching index value calculation unit
108 Graph selection unit
110 Machine learning model
T1 Explanation result information
T2 Protein position information

**Claims**

1. An Information processing program causing a computer to perform a process comprising:

   in classification processing on input graph structure data using a machine learning model (110), acquiring a contribution degree in the classification processing for each of a plurality of partial regions included in graph structure data; and
   determining an evaluation for the machine learning model based on similarity between the contribution degree and designation information for the partial region of the graph structure data.

2. The information processing program according to claim 1, wherein the process further comprises:
   calculating a matching index value indicating the similarity by calculating a ratio of a sum of contribution degrees corresponding to partial regions specified by the designation information among all the partial regions included in the graph structure data to a sum of contribution degrees corresponding to all the partial regions included in the graph structure data.

3. The information processing program according to claim 2, wherein the process further comprises:
   calculating an evaluation value for the machine learning model based on a sum of the matching index value and a classification accuracy degree of the machine learning model.

4. The information processing program according to claim 3, wherein
   weights are set for the matching index value and the classification accuracy degree.

5. An information processing device comprising a controller, the controller being configured to execute a process comprising:

   in classification processing on input graph structure data using a machine learning model, acquiring a contribution degree in the classification processing for each of a plurality of partial regions included in graph structure data; and
   determining an evaluation for the machine learning model based on similarity between the contribution degree and designation information for the partial region of the graph structure data.

6. The information processing device according to claim 5, wherein
the process further comprising:
calculating a matching index value indicating the similarity by calculating a ratio of a sum of contribution degrees corresponding to partial regions specified by the designation information among all the partial regions included in the graph structure data to a sum of contribution degrees corresponding to all the partial regions included in the graph structure data.

7. The information processing device according to claim 6, wherein
the process further comprising:
calculating an evaluation value for the machine learning model based on a sum of the matching index value and a classification accuracy degree of the machine learning model.

8. The information processing device according to claim 7, wherein
weights are set for the matching index value and the classification accuracy degree.

9. A computer-implemented information processing method comprising:

in classification processing on input graph structure data using a machine learning model, acquiring a contribution degree in the classification processing for each of a plurality of partial regions included in graph structure data; and determining an evaluation for the machine learning model based on similarity between the contribution degree and designation information for the partial region of the graph structure data.

10. The computer-implemented information processing method according to claim 9, comprising:
calculating a matching index value indicating the similarity by calculating a ratio of a sum of contribution degrees corresponding to partial regions specified by the designation information among all the partial regions included in the graph structure data to a sum of contribution degrees corresponding to all the partial regions included in the graph structure data.

11. The computer-implemented information processing method according to claim 10, comprising:
calculating an evaluation value for the machine learning model based on a sum of the matching index value and a classification accuracy degree of the machine learning model.

12. The computer-implemented information processing method according to claim 11, wherein
weights are set for the matching index value and the classification accuracy degree.

## FIG.1

# FIG.2

# FIG.3

| Node ID | Prediction Contribution Degree |
|---|---|
| 1 | 0.2 |
| 2 | 0.1 |
| 3 | 0.8 |
| 4 | 0.9 |
| 5 | 0.8 |
| 6 | 0.1 |
| 7 | 0.2 |
| 8 | 0.6 |
| 9 | 0.1 |

T1

(It Is Assumed That Full Length Is 9 as Example)

# FIG.4

| Position |
|:--------:|
| 3 |
| 4 |
| 5 |
| 8 |

← T2

# FIG.5

T2:Protein Position Information

| Position |
|---|
| 3 |
| 4 |
| 5 |
| 8 |

$$\frac{\text{Sum of Prediction Contribution Degrees of Positions Indicated in Protein Position Information}}{\text{Sum of Prediction Contribution Degrees of All Positions}}$$

⇩

$$\frac{\text{Sum of Prediction Contribution Degrees of Node IDs. 3, 4, 5, and 8}}{\text{Sum of Prediction Contribution Degrees of Node IDs. 1 to 9}}$$

⇩

$$\frac{\text{Sum of Prediction Contribution Degrees of Node IDs. 3, 4, 5, and 8} = 0.8+0.9+0.8+0.6 = 3.1}{\text{Sum of Prediction Contribution Degrees of Node IDs. 1 to 9} = 0.2+0.1+0.8+0.9+0.8+0.1+0.2+0.6+0.1 = 3.8} = 0.816$$

⇩

0.816

Matching Index Value

It Is Indicated Whether High Prediction Contribution Degrees Are Concentrated at Nodes Indicated in Protein Position Information T2

| Node ID | Prediction Contribution Degree |
|---|---|
| 1 | 0.2 |
| 2 | 0.1 |
| 3 | 0.8 |
| 4 | 0.9 |
| 5 | 0.8 |
| 6 | 0.1 |
| 7 | 0.2 |
| 8 | 0.6 |
| 9 | 0.1 |

| Node ID | Prediction Contribution Degree |
|---|---|
| 1 | 0.2 |
| 2 | 0.1 |
| 3 | 0.8 |
| 4 | 0.9 |
| 5 | 0.8 |
| 6 | 0.1 |
| 7 | 0.2 |
| 8 | 0.6 |
| 9 | 0.1 |

(It Is Assumed That Full Length Is 9 as Example)

T1:Explanation Result Information

# FIG.6

# FIG.7

```
                    ┌─────────────┐
                    │    START    │
                    └─────────────┘
                           │
                           ▼
        ┌──────────────────────────────────┐
        │     Acquire Protein Position      │  S1
        │   Information and Explanation     │
        │        Result Information         │
        └──────────────────────────────────┘
                           │
                           ▼
        ┌──────────────────────────────────┐
        │     Calculate Matching Index      │  S2
        │      Value Based on Protein       │
        │       Position Information and    │
        │   Explanation Result Information  │
        └──────────────────────────────────┘
                           │
                           ▼
        ┌──────────────────────────────────┐
        │  Calculate Evaluation Value for   │  S3
        │  Machine Learning Model Based     │
        │   on Matching Index Value and     │
        │  Classification Accuracy Degree   │
        └──────────────────────────────────┘
                           │
                           ▼
        ┌──────────────────────────────────┐
        │   Evaluate Machine Learning       │  S4
        │    Models (Graphs) Based on       │
        │  Plurality of Evaluation Values   │
        └──────────────────────────────────┘
                           │
                           ▼
                    ┌─────────────┐
                    │     END     │
                    └─────────────┘
```

## INTERNATIONAL SEARCH REPORT

| International application No. |
| --- |
| **PCT/JP2023/033775** |

**A. CLASSIFICATION OF SUBJECT MATTER**

*G06N 20/00*(2019.01)i
FI:  G06N20/00

According to International Patent Classification (IPC) or to both national classification and IPC

**B. FIELDS SEARCHED**

Minimum documentation searched (classification system followed by classification symbols)

G06N20/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | US 2021/0166779 A1 (DEEPMIND TECHNOLOGIES LIMITED) 03 June 2021 (2021-06-03) entire text | 1-12 |
| A | WO 2008/062906 A1 (IN-SILICO SCIENCES, INC.) 29 May 2008 (2008-05-29) entire text | 1-12 |
| A | JP 2022-535769 A (RUBRYC THERAPEUTICS, INC.) 10 August 2022 (2022-08-10) entire text | 1-12 |
| A | US 11176462 B1 (RO5 INC.) 16 November 2021 (2021-11-16) entire text | 1-12 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **20 November 2023** | **28 November 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2023/033775**

| Patent document cited in search report | | | Publication date (day/month/year) | Patent family member(s) | | | Publication date (day/month/year) |
|---|---|---|---|---|---|---|---|
| US | 2021/0166779 | A1 | 03 June 2021 | CN | 114503203 | A | |
| WO | 2008/062906 | A1 | 29 May 2008 | US | 2010/0057420 | A1 | |
| JP | 2022-535769 | A | 10 August 2022 | US | 2021/0166788 | A1 | |
| | | | | KR | 10-2022-0039659 | A | |
| | | | | CN | 114401734 | A | |
| US | 11176462 | B1 | 16 November 2021 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019159432 A
- US 11176462 B
- WO 2018052131 A
- JP 2022536343 A
- JP 2018506963 A
- US 20220076783